(19) Europäisches Patentamt
European Patent Office
Office européen des brevets

(11) EP 2 601 511 B1

(12) # EUROPÄISCHE PATENTSCHRIFT

(45) Veröffentlichungstag und Bekanntmachung des Hinweises auf die Patenterteilung:
**11.07.2018 Patentblatt 2018/28**

(21) Anmeldenummer: **11746464.4**

(22) Anmeldetag: **01.08.2011**

(51) Int Cl.:
***G01N 17/02*** (2006.01) ***G01N 33/18*** (2006.01)

(86) Internationale Anmeldenummer:
**PCT/EP2011/003842**

(87) Internationale Veröffentlichungsnummer:
**WO 2012/016673 (09.02.2012 Gazette 2012/06)**

(54) **VERFAHREN ZUR BESTIMMUNG DER NEIGUNG ZUR VEROCKERUNG UND SYSTEM HIERFÜR**

METHOD FOR DETERMINING THE TENDENCY TO IRON DEPOSITION AND SYSTEM THEREFOR

PROCÉDÉ DE DÉTERMINATION DE LA PROPENSION À LA FORMATION D’OCRE FERREUX ET SYSTÈME CORRESPONDANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.08.2010 DE 102010033056**

(43) Veröffentlichungstag der Anmeldung:
**12.06.2013 Patentblatt 2013/24**

(73) Patentinhaber: **Leuphana Universität Lüneburg Stiftung Öffentliche**
**21335 Lüneburg (DE)**

(72) Erfinder:
- **OPEL, Oliver**
  **21337 Lüneburg (DE)**
- **RUCK, Wolfgang, K., L.**
  **21391 Reppenstedt (DE)**

(74) Vertreter: **Kröncke, Rolf et al**
**Gramm, Lins & Partner**
**Patent- und Rechtsanwälte PartGmbB**
**Freundallee 13 a**
**30173 Hannover (DE)**

(56) Entgegenhaltungen:
- **CHARLES A CRAVOTTA: "Passive aerobic treatment of net-alkaline, iron-laden drainage from a flooded underground anthracite mine, Pennsylvania, USA", MINE WATER AND THE ENVIRONMENT ; JOURNAL OF THE INTERNATIONAL MINE WATER ASSOCIATION (IMWA), SPRINGER, BERLIN, DE, Bd. 26, Nr. 3, 18. August 2007 (2007-08-18), Seiten 128-149, XP019521525, ISSN: 1616-1068, DOI: 10.1007/S10230-007-0002-8**
- **HOVE ET AL: "Mechanisms of formation of iron precipitates from ferrous solutions at high and low pH", CHEMICAL ENGINEERING SCIENCE, OXFORD, GB, Bd. 63, Nr. 6, 19. November 2007 (2007-11-19), Seiten 1626-1635, XP022453537, ISSN: 0009-2509, DOI: 10.1016/J.CES.2007.11.016**
- **MORGAN ET AL: "The effect of pH on the kinetics of spontaneous Fe(II) oxidation by O2 in aqueous solution - basic principles and a simple heuristic description", CHEMOSPHERE, PERGAMON PRESS, OXFORD, GB, Bd. 68, Nr. 11, 27. Juli 2007 (2007-07-27) , Seiten 2080-2084, XP022192406, ISSN: 0045-6535, DOI: 10.1016/J.CHEMOSPHERE.2007.02.015**
- **H. Prange: "Handout zur Projektarbeit 7. Sem. Umweltbiologie (ISTAB), Hochschule Bremen WS 04/05", , 1. Januar 2005 (2005-01-01), XP55009517, Gefunden im Internet: URL:http://www.wuemme-meerforelle.de/resources/Handout-OCKER.pdf [gefunden am 2011-10-13]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 601 511 B1

- WERNER STUMM ET AL: "Oxygenation of Ferrous Iron", INDUSTRIAL & ENGINEERING CHEMISTRY, Bd. 53, Nr. 2, 1. Februar 1961 (1961-02-01), Seiten 143-146, XP55009503, ISSN: 0019-7866, DOI: 10.1021/ie50614a030
- F MILLERO ET AL: "The oxidation kinetics of Fe(II) in seawater", GEOCHIMICA ET COSMOCHIMICA ACTA, Bd. 51, Nr. 4, 1. April 1987 (1987-04-01), Seiten 793-801, XP55009559, ISSN: 0016-7037, DOI: 10.1016/0016-7037(87)90093-7
- Fachbeitrag Opel ET AL: "Relationships Between Measured and Calculated Reduction- Oxidation Potentials in Groundwater from Aquifer Thermal Energy Storage (Heat and Cold Storage) at the Berlin Reichstag Site (Germany)", Vom Wasser, 1 January 2008 (2008-01-01), pages 3-38, XP55158027, Retrieved from the Internet: URL:- [retrieved on 2014-12-11]

## Beschreibung

Die vorliegende Anmeldung betrifft ein Verfahren zur Bestimmung der Bildungsrate von $Fe^{3+}$ und/oder $Fe^{3+}$-Hydroxid in Wasser oder wässrigen Fluiden umfassend die Bestimmung des Redoxpotentials $Fe^{2+}/Fe^{3+}$ mit Hilfe einer Redoxelektrode, die Bestimmung des pH-Werts, die Bestimmung der Temperatur und die Bestimmung des Sauerstoffgehaltes. Mit Hilfe des erfindungsgemäßen Verfahrens ist es möglich die Verockerungsrate von Wasser bzw. wässrigen Fluiden zu bestimmen. Weiterhin wird ein System zur Bestimmung der Verockerungsrate bereitgestellt, insbesondere ein System ausgebildet als Sensor.

### Stand der Technik

Verockerung ist die Bildung von voluminösen eisenhaltigen Ausfällungen in wässrigen Systemen. Unter Verockerung wird vorliegend die Bildung von $Fe(OH)_3$ verstanden. Die Verockerung tritt aufgrund der Oxidation in Wasser gelösten Eisens durch Sauerstoff auf. Dabei wird in Wasser gelöstes Eisen (II) zu Eisen (III) oxidiert. Das oxidierte Eisen (III) bildet mit im Wasser enthaltenen Hydroxidionen unter Bildung von Säure (Hydroniumionen) unlösliche, voluminöse, braune Eisenhydroxid ($Fe(OH)_3$)-Niederschläge. Solche Niederschläge können sowohl in sogenannten offenen Systemen aber auch in geschlossenen Systemen auftreten. Unter offenen Systemen werden insbesondere offene hydraulische System verstanden, die natürliche (Grund-)Wässer nutzen. Beispielhaft sind hier Förder- und Schluckbrunnen, andere Anlagen zur Trinkwassergewinnung und -aufbereitung, Leitungssysteme, Systeme zur Energiegewinnung, Kühlung von Gebäuden und Wärmespeicherung mit Nutzung natürlicher Gewässer genannt.

Unter geschlossenen Systemen wird verstanden, dass ein solches System nicht im stofflichen sondern z.B. im energetischen Austausch mit der Umgebung steht. Beispiele hierfür sind Heizungsanlagen, Anlagen zur Wärmespeicherung, Anlagen zur Gebäude- oder Gerätekühlung oder Vorrichtungen zum Spülen eines Gerätes oder einer Anlage.

Verockerungen, Korrosion und Rostbildung durch im Wasser oder wasserhaltigen Fluid gelösten Eisen oder durch Korrosionsprozesse, die zu einer Freisetzung von Eisen in das Wasser oder wasserhaltigem Fluid führen, können in beiden Systemen auftreten. Sie führen zum Zusetzen von Leitungen und Filtern, einer Blockierung von Ventilen und Pumpen sowie einer Kapazitätsminderung von Förder- und Schluckbrunnen und in der Regel zu steigendem Energiebedarf für den Betrieb der betreffenden Anlage. Verockerung erfordert Wartungsmaßnahmen, wie Reinigungen, Brunnenregenerationen, Austausch defekter Bauteile, Ventile, Pumpen, etc. und Stillstandszeiten sowie häufig auch über längere Zeiträume unbemerkte Leistungsverringerungen und beispielsweise erhöhtem Energiebedarf in Heizungssystemen. Je nach Anlagentyp handelt es sich um Störungen, die oft unbemerkt auf Anlagendefekte zurückgehen, oder um üblicherweise auftretende, jedoch bereits im Routinebetrieb nicht genau vorhersehbare Ereignisse, die durch unbemerkte Anlagendefekte plötzlich oder eher als erwartet eintreten können. Die Verockerung ist ein seit langem bekanntes Problem in der Trinkwassergewinnung und Wasseraufbereitung sowie beim Einsatz von Wasser bzw. wasserhaltigen Fluiden oder beim Einsatz von wasserhaltigen Fluiden in Verbindung mit eisenhaltigen Werkstoffen. Die entstehenden braunen Niederschläge (Verockerung, Rost) sind darüber hinaus aus hygienischen Gründen unerwünscht, so dass insbesondere in der Trinkwassergewinnung und Wasseraufbereitung eine Verockerung vermieden werden sollte.

Die Kinetik der zugehörigen Oxidationsreaktion ist komplex. Während die Oxidation in der wässrigen Phase in künstlichen Systemen sowie in der wässrigen Phase natürlicher Systeme verhältnismäßig gut beschrieben werden kann, sind Oxidationsraten in realen Systemen derzeit nicht vorhersehbar. Dies hat verschiedene Gründe. Zum einen muss das Wasser in seiner Zusammensetzung sowie die Wirkung einzelner Wasserinhaltsstoffe auf die Aktivität der Eisenionen für die Berechnung aus wasseranalytischen Daten vollständig bekannt sein. Dies ist im realen System in den seltensten Fällen der Fall. Insbesondere bei der Wirkung einzelner Wasserinhaltsstoffe auf die Eisenionenaktivität - z.B. durch Komplexierung oder die Funktion einiger Substanzen als Elektronenshuttle - sowie evtl. gegenseitiger Beeinflussung der Wasserinhaltsstoffe untereinander bei der Wirkung auf Eisenionen besteht großes Unwissen. So haben die Erfinder selbst vor kurzem in einer Arbeit äußerst starke, bis dahin unbekannte Einflüsse auf gelöste Eisenionen durch die Bildung eines Hydroxokarbonatkomplexes in realen Wässern identifiziert (Opel, O., et al., 2008, Vom Wasser 106(4), 14-21). Bisherige Arbeiten beruhen in der Regel nur auf einfachen künstlichen Systemen. So wird in Cravotta, 2007, Mine Water Environ, 26, 128-149 ein Verfahren beschrieben, dass unter anderem die Eisenoxidation zum Thema hat. Die Oxidation von $Fe^{2+}$ wird mit dem Programm PHREEQC untersucht.

Zum anderen spielen Mikroorganismen eine nicht zu unterschätzende Rolle. In aus Brunnenregenerationen gewonnenen Eisenschlammproben, aber auch in Rohrleitungen werden gewöhnlich eisenoxidierende Organismen gefunden. In einer Reihe von Veröffentlichungen wurde der Nachweis erbracht, dass eisenoxidierende Organismen in der Lage sind, effektiv die Oxidation von Eisen (II) zu Eisen (III) obligat zur Energiegewinnung zu nutzen bzw. fakultativ zu katalysieren. In der Folge ist der Einfluss eisenoxidierender Organismen auf die Verockerung von Brunnen zur Grundwasserförderung unbestritten. Auch bei der Mobilisation von oxidierten Eisendepots spielen Mikroorganismen eine bedeutende Rolle. Hierbei ist neben Abhängigkeiten von Sauerstoffpartialdruck und pH-Wert die Katalyse der abiotischen Oxidationen durch bereits gebildetes Eisen (III)-Hydroxid von besonderer Bedeutung, wobei biogene $Fe(OH)_3$-Schlämme

im Allgemeinen aufgrund der Umhüllung des biotisch gebildeten $Fe(OH)_3$ mit extrazellulärer Substanz spezifisch eine geringere Aktivität bzgl. ihrer katalytischen Wirkung aufweisen als abiotisch gebildetes Eisen (III)-Hydroxid. Diese Einflüsse sind jedoch bislang nicht quantitativ beschrieben worden. Dies gilt ebenso für die eigentliche Kinetik biotischer Eisenoxidation unter nicht idealen, realistischen Bedingungen. Es ist jedoch bekannt, dass die biotische Oxidation durch Mikroorganismen in Abhängigkeit des pH-Werts einen relativ geringen bzw. abschätzbaren Einfluss aufzeigen. Als weitere Einflussgrößen sei z.B. die oberflächenkatalysierte Oxidation genannt, ohne dass diese bisher näher quantifiziert wurden.

Im Stand der Technik ist somit kein Verfahren zur Bestimmung der Bildungsrate von $Fe^{3+}$ und/oder $Fe^{3+}$-Hydroxid in Wasser oder in wasserhaltigen Fluiden zur Vorhersage des Verockerungspotentials beschrieben. Bisherige Lösungsversuche zur Vermeidung der Ausbildung der Eisenniederschläge durch Oxidation von gelöstem Eisen (II) mit Sauerstoff sind unter anderem die folgenden. In geschlossenen hydraulischen Systemen werden Korrosionsinhibitoren eingesetzt, die umweltschädlich sind. Dies hat in einigen Fällen genehmigungsrechtliche Konsequenzen. In anderen Fällen wird das Risiko durch mögliche Leckagen in Kauf genommen. Anlagentechnisch können Korrosionsschutzmittel durch biotische und abiotische Prozesse zunächst unbemerkt ihre Funktion verlieren und unwirksam werden, was in einem solchen Fall Anlagenschäden hervorruft. Solche Korrosionsschutzmittel können natürlich in offenen Systemen, wie bei der Grundwassergewinnung und Trinkwasserproduktion, nicht eingesetzt werden. Hier wird versucht das Einbringen von Luftsauerstoff möglichst gering zu halten, um ein entsprechendes Oxidieren und somit Ausfällen des Eisenhydroxids zu vermeiden. In der Praxis gelingt dies aber nicht vollständig. Bei besonders eisenhaltigen Gewässern muss in der Regel auf eine Nutzung verzichtet werden.

Weiterhin besteht das Risiko des unbemerkten Eindringens von Sauerstoff durch poröse Dichtungen oder andere Veränderungen der Wasserchemie, die die Verockerungsneigung der Wässer zunächst unbemerkt erhöhen können. Dies führt in solchen Fällen zu einer vorzeitigen Beeinträchtigung z.B. der Pumpenleistung einhergehend mit erhöhtem Energieverbrauch für die Förderung der Wässer. Filter können sich vorzeitig zusetzen. Auch in der Trinkwasseraufbereitung bzw. Grundwasserreinigung eingesetzte Systeme können durch Bildung von Niederschlägen, d.h. durch Verockerung, zugesetzt werden.

Die Verockerung von Förder- und Schluckbrunnen sowie Filteranlagen kann bei der Nutzung/ Handhabung natürlicher Gewässer im Allgemeinen nicht verhindert werden. Allerdings besteht ein Bedürfnis darin, die Wartungsintervalle dieser Systeme möglichst genau zu kennen, um eine entsprechende Verockerung rechtzeitig zu bekämpfen. Bisherige Wartungsintervalle beruhen auf Erfahrungsdaten und sind für jeden Brunnen individuell. Da die Wartungen aber hohe Kosten erzeugen und das System meist zur Wartung außer Betrieb genommen werden muss, besteht großes Interesse daran, die Verockerungsrate zu bestimmen, um die Wartung so rechtzeitig durchzuführen, ohne die Systeme selbst zu schädigen. Bisher werden in der Einfahrphase eines Brunnens die notwendigen Wartungsintervalle durch Trial-and-Error-Verfahren erarbeitet. Eine plötzliche Veränderung der Wasserchemie oder Eintreten von Sauerstoff aufgrund eines Defektes verändert aber die notwendigen Wartungsintervalle. In der Praxis wird die Brunnenleistung beobachtet und der Brunnen bei plötzlichem Eintreten einer Verschlechterung bzw. von sichtbaren Verockerungserscheinungen außerhalb der geplanten Wartungsintervalle regeneriert. Diese Wartungen sind allerdings nicht vorhersehbar, sondern müssen dann kurzfristig durchgeführt werden. Durch eine rechtzeitige Wartung kann das Zusetzen und ggf. der vollständige Verlust eines Brunnens, inklusive des dann erforderlichen kostenaufwendigen Rückbaus, bzw. analoge, durch Eisenhydroxidbildung und Korrosion/Rostbildung hervorgerufene Schäden an hydraulischen Anlagen verhindert werden. Eine Überprüfung der Wasserchemie in kurzen Intervallen, um eine Veränderung des Wassers frühzeitig zu erkennen, ist derzeit allerdings aufgrund der kostenintensiven und dazu notwendigen umfangreichen Laboruntersuchungen sowie des notwendigen Wissens der Wirkung von Wasserinhaltsstoffen auf die Aktivität der Eisenionen bislang de facto nicht umsetzbar.

Es besteht daher ein großer Bedarf, die Bildungsrate von Eisen (III) Ionen und Eisen (III)-Hydroxid und somit die Verockerungsrate und Korrosion/Rostbildung in Wasser bzw. wasserhaltigen Fluiden z.B. in hydraulischen Systemen bevorzugt kontinuierlich zu überwachen.

Auf der Seite der elektrochemischen Sensorik stellen die pH-Glaselektrode sowie die Clark-Sauerstoffelektrode seit langem angewandte und gut erforschte Messmethoden dar. Es werden jeweils die Aktivitäten der entsprechenden Spezies gemessen, d.h. das Maß der chemischen Verfügbarkeit bzw. Reaktionsfähigkeit der gemessenen Spezies ($H^+$ bzw. $O_2$). Im Falle von Sauerstoff ist dies der Partialdruck in Lösung. Beide Parameter sind von außerordentlicher Bedeutung für die Oxidation von gelöstem Eisen. Dabei steigt die Reaktionsgeschwindigkeit mit Sauerstoff linear, fällt mit $H^+$ jedoch kontral ab (bzw. steigt quadratisch mit der OH-Konzentration an). Die Temperatur als Eingangsparameter stellt ebenso kein messtechnisches Problem dar.

Zur Messung von Eisen stehen demgegenüber keine elektrochemischen Methoden am Markt zur Verfügung, die die für ein Handgerät bzw. Festinstallation wünschenswerten Eigenschaften Robustheit, Stabilität, geringer Chemikalienverbrauch und Kompaktheit vereinen. Heutzutage wird Eisen zumeist im Labor photometrisch oder mittels spektroskopischer Methoden in seiner relevanten zweiten Oxidationsstufe bestimmt. Als elektrochemische Methode steht die Polarometrie zur Verfügung. Die bestimmte Größe ist hierbei zunächst jedoch nicht die Aktivität der Eisen (II)-Ionen,

sondern die Konzentration. In der Literatur sind elektrochemische Eisenelektroden auf Basis von Chalkogenidmembranen beschrieben, die jedoch nur für Eisen (III)-Elektroden sensitiv sind und Probleme mit der Anwendung Stabilität haben. Ein Eisen (II)-Sensor auf der Basis von Chalkogenidgläsern ist theoretisch denkbar. Dieser Sensor würde aber die Eisenaktivität direkt messen, die Nachteile überwiegen jedoch durch starke Signaldrift, Leaching der Chalkogenidmembranen bekannte Querempfindlichkeiten und eine schlechte Anwendbarkeit insbesondere für Onlinemessgeräte sowie eine ungünstige Handhabung gegenüber etwaigen Vorteilen.

Hinsichtlich der Anwendbarkeit von Redoxpotentialmessungen für die Detektion von Eisen (II)-induzierten Wässer ist dieses z.B. in Opel, O., et al., 2008, Vom Wasser 106(4), 14-21 dargestellt. Im Wesentlichen wurde die Rückführung von gemessenen Redoxpotentialen auf wasseranalytischen Daten seit der Erfindung der Redoxelektrode im Jahr 1920 mehrfach erfolglos versucht. Es wurde festgestellt, dass dieses in vielen Fällen nicht möglich ist. Als Gründe wurden die Ausbildung von Mischpotentialen, die schlechte Reaktivität der meisten Ionenspezien an der Elektrode sowie die Veränderung der Platinelektrodenoberfläche durch Sauerstoff und Sulfide genannt. Selbst die Verwendbarkeit des mittels Redoxelektrode gemessenen Redoxpotentials als operationaler, zu Vergleichzwecken zu nutzender Parameter, wurde aus diesen Gründen in Frage gestellt. Opel, O. et al. stellt dabei als erster eine Verknüpfung gemessener, mit aus Eisengehalten berechneten Redoxpotentialen im Bereich der Erklärung gemessener Redoxpotentiale dar.

Aufgabe der vorliegenden Erfindung ist somit die Bereitstellung von Verfahren sowie Vorrichtungen und Systemen zur Bestimmung der Bildungsrate von $Fe^{3+}$ und/oder $Fe^{3+}$-Hydroxid im Wasser oder wasserhaltigen Fluiden, um das Verockerungspotential bzw. die Verockerungsrate in diesem Wasser bzw. wasserhaltigen Fluiden zu bestimmen und zu überwachen. Dadurch kann rechtzeitig die Wartung der betroffenen Systeme erfolgen und die Wartungsintervalle einfach bestimmt werden.

<u>Beschreibung der Erfindung</u>

Die Erfinder haben nun erfolgreich ein Verfahren sowie eine Vorrichtung bzw. ein System entwickelt, das eine schnelle und vor allem preiswerte kompakte Analyse zu der Verockerungsrate bzw. Verockerungsneigung eines Wassers, wässrigen Fluids, insbesondere eines wasserhaltigen Systems ohne aufwendige Laboruntersuchungen erlaubt.
Das erfindungsgemäße Verfahren erlaubt dabei eine frühzeitige Erkennung einer zunehmenden Verockerungsneigung der Wässer bzw. wasserhaltigen Fluide und entsprechender wasserhaltiger Systeme, so dass auch bereits im Betrieb eine frühzeitige Veränderung bzw. Zunahme der Verockerungsneigung oder von Korrosionsprozessen erkannt werden kann oder eine Beurteilung von Wasserqualitäten hinsichtlich der Verockerungsneigung vor der Planung von Anlagen möglich ist.
D.h. das erfindungsgemäße Verfahren und die erfindungsgemäßen Vorrichtungen und Systeme erlauben ein Erkennen der Verockerung vor dem Verockerungsereignis selbst, um so entsprechende, präventive Maßnahmen, wie Wartung der Systeme oder Austausch entsprechender Bauteile, durchzuführen. Dadurch können Einspar- und Optimierungspotentiale im Betrieb sowie eine erhöhte Betriebssicherheit erreicht werden.
Das erfindungsgemäße Verfahren zur Bestimmung der Bildungsrate von $Fe^{3+}$ und/oder $Fe^{3+}$-Hydroxid in Wasser oder wasserhaltigen Fluiden ist ein Verfahren entsprechend dem unabhängigen Verfahrensanspruch 1. In einer bevorzugten Ausführungsform handelt es sich bei dem Verfahren um eines weiterhin umfassend mindestens einen der Schritte

- Bestimmen der $Fe^{2+}$ Konzentration,
- Bestimmen der $Fe^{3+}$ Konzentration,
- Bestimmen der $Fe^{2+}$ Aktivität,
- Bestimmen des $CO_2$-Gehalts, und/oder
- Bestimmen der Leitfähigkeit des Wassers oder wässrigen Fluids.

Das erfindungsgemäße Verfahren kann zusätzlich die Bestimmung des Gehaltes an Nitrat, Phosphat, organischem Kohlenstoff und/oder Karbonat beinhalten, die Bestimmung der Turbidität und/oder die Bestimmung der Färbung. Erfindungsgemäß konnte gezeigt werden, dass durch Bestimmung der Bildungsrate von $Fe^{3+}$ und/oder $Fe^{3+}$-Hydroxid in Wasser oder im wässrigen Fluid die Verockerungsneigung des Wassers bzw. des wässrigen Fluids einfach bestimmt werden kann. Mit Hilfe der Eisenionenaktivität bestimmt durch das Redoxpotential von $Fe^{2+}$/ $Fe^{3+}$, der Bestimmung des pH-Wertes, der Temperatur und des Sauerstoffgehaltes des Wassers, kann einfach die Bildungsrate von $Fe^{3+}$ und/oder $Fe^{3+}$-Hydroxid bestimmt werden.
Die Aktivität ist dabei gegenüber der Konzentration der Eisenionen die für die Geschwindigkeit der zur Verockerung führenden Eisenionen ausschlaggebende Messgröße. Die Einflüsse auf die Oxidationsgeschwindigkeit, die durch komplexierende, hemmende oder beschleunigende, als Elektronenshuttle wirkende Wasserinhaltsstoffe zustande kommen, werden mit ihrer Wirkung miterfasst, ohne eine aufwendige Wasseranalytik und eine genaue Kenntnis des Vorgangs zu benötigen.

Die Verockerungsneigung kann daher einfach bestimmt und ggf. als Verockerungspotential mit Hilfe einer Skala dargestellt werden. Z.B. kann durch logarithmische Darstellung einer einheitslosen Skala das Verockerungsrisiko einfach dem Anwender dargestellt werden.

Das erfindungsgemäße Verfahren erlaubt durch die einfache Bestimmung der einzelnen Parameter eine kontinuierliche und/oder Echtzeitbestimmung des Verockerungsrisikos des Wasser oder der wässrigen Fluiden insbesondere in einem geschlossenen wasserhaltigen System und somit eine permanente vor-Ort Überwachung. Aufwendige Laboruntersuchungen sind nicht notwendig. Vielmehr kann die Bestimmung des Verockerungsrisikos präventiv die Wartung der wasserhaltigen Systeme erfolgen, ohne dass eine dauerhafte Schädigung dieses Systems erfolgt.

Das erfindungsgemäße Verfahren ist daher insbesondere eines zur Bestimmung des Verockerungsrisikos in offenen und geschlossenen, wasserhaltigen Systemen. Solche offenen und geschlossenen, wasserhaltigen Systeme schließen die folgenden ein: Förder- und Schluckbrunnen, Anlagen zur Trink- und Brauchwassergewinnung, Leitungssysteme, geothermische Anlagen, Heizungsanlagen, Anlagen zur Wärmespeicherung, Anlagen zur Gebäude- und Gerätekühlung oder Vorrichtungen zum Spülen von Geräten oder Anlagen.

Das erfindungsgemäße Verfahren ist in einer bevorzugten Ausführungsform eines, das das Verockerungsrisiko darlegt, wobei ein Verockerungsrisiko z.B. sehr groß ist, wenn die Bildungsrate von $Fe^{3+}$ und/oder $Fe^{3+}$-Hydroxid $\geq 0{,}1$ $g/m^{3*}min$ ist, das Verockerungsrisiko groß ist, wenn die Bildungsrate vn $Fe^{3+}$ und/oder $Fe^{3+}$-Hydroxid < 0,1 $g/m^{3*}min$ und $\geq 0{,}01$ $g/m^{3*}min$ ist, es durchschnittlich ist, wenn die Bildungsrate von $Fe^{3+}$ und/oder $Fe^{3+}$-Hydroxid < 0,01 $g/m^{3*}min$ und $\geq 0{,}001$ $g/m^{3*}min$ ist und das Verockerungsrisiko gering ist, wenn die Bildungsrate von $Fe^{3+}$ und/oder $Fe^{3+}$-Hydroxid < 0,001 $g/m^{3*}min$ ist.

Die Bestimmung der einzelnen Parameter kann dabei mit bekannten Vorrichtungen und Einrichtungen erfolgen. Die Bestimmung des Redoxpotentials $Fe^{2+}$/ $Fe^{3+}$ erfolgt z.B. mit Hilfe einer Redoxelektrode, wie einer Einstabmaßkette. Eine Einstabmaßkette ist eine Elektrode mit Platin- oder Goldelektrode und Referenzelektrode als eine Einheit.

Die Bestimmung des pH-Werts kann mit üblichen Mitteln geschehen, wie z.B. einer pH-Glaselektroden-Einstabmesskette.

Die Temperatur wird mit einer entsprechenden Einrichtung bestimmt. Die Bestimmung des Sauerstoffgehalts erfolgt z.B. mit Hilfe von Platin-Temperatur-Flachmesswiderständen (Pt-Elemente).

Das erfindungsgemäße Verfahren ist insbesondere geeignet, die Bildungsrate von $Fe^{3+}$ und/oder $Fe^{3+}$-Hydroxid und somit ein Verockerungsrisiko bzw. die Verockerungsrate in Wasser bzw. im wässrigen Fluid zu bestimmen, wenn dieses als Arbeitsmedium eines hydraulischen Systems z.B. bei einem Brunnen verwendet wird, aber auch in Anlagen zur Trink- oder Brauchwassergewinnung, in Leitungssystemen, Heizungsanlagen, Anlagen zur Wärmespeicherung, Anlagen zur Gebäude- oder Gerätekühlung oder eine Vorrichtung zur Spülung eines Geräts oder einer Anlage.

Vorliegend wird unter dem Ausdruck "hydraulisches System" eines verstanden, das Wasser bzw. ein wässriges Fluid beinhaltet. Bevorzugte hydraulische Systeme sind die hierin genannten offenen und geschlossenen Systeme.

Unter Verockerung wird vorliegend die Bildung von $Fe^{3+}$-Hydroxid verstanden. Entsprechend ist die Bildungsrate von $Fe^{3+}$ bzw. $Fe^{3+}$-Hydroxid ein direkter Indikator für das Verockerungsrisiko. Dieses Risiko kann zur besseren Erfassung bzw. Darstellung logarithmisch dargestellt werden.

Wässrige Fluide sind Flüssigkeiten, die überwiegend, also über 50% aus Wasser ($H_2O$) bestehen.

Unter Wässer werden vorliegend weiterhin sowohl stehende als auch fließende, offene oder geschlossene Gewässer verstanden.

In einem weiteren Aspekt richtet sich die vorliegende Anmeldung auf eine Vorrichtung oder ein System zur Bestimmung der Bildungsgeschwindigkeit von $Fe^{3+}$ und/oder $Fe^{3+}$-Hydroxid in Wasser oder wässrigen Fluiden umfassend eine Einrichtung zur Bestimmung des Redoxpotenzials von $Fe^{2+}$/ $Fe^{3+}$, die eine Redoxelektrode umfasst; eine Einrichtung zur Bestimmung des Sauerstoffgehalts im Wasser oder wässrigen Fluid; eine Einrichtung zur Bestimmung der Temperatur des Wassers; und eine Einrichtung zur Bestimmung des pH-Werts des Wassers oder wässrigen Fluids. Das erfindungsgemäße System ist ausgebildet zur Durchführung des erfindungsgemäßen Verfahrens.

Dem Fachmann sind entsprechende Einrichtungen zur Bestimmung des Redoxpotentials von $Fe^{2+}$/ $Fe^{3+}$, zur Bestimmung des Sauerstoffgehalts in Wasser, zur Bestimmung der Temperatur des Wassers und zur Bestimmung des pH-Werts des Wassers bekannt.

In einer bevorzugten Ausführungsform der erfindungsgemäßen Vorrichtung und des erfindungsgemäßen Systems umfasst diese mindestens weiterhin eine Einrichtung zur Bestimmung eines der folgenden Parameter: $Fe^{2+}$-Konzentration, $Fe^{2+}$-Aktivität, $CO_2$-Konzentration, Leitfähigkeit, oder $Fe^{3+}$-Konzentration des Wassers bzw. des wässrigen Fluids.

In einer besonders bevorzugten Ausführungsform umfasst das erfindungsgemäße System oder die erfindungsgemäße Vorrichtung weiterhin eine Auswerteeinheit zum Bestimmen der Bildungsgeschwindigkeit von $Fe^{3+}$ und/oder $Fe^{3+}$-Hydroxid in Wasser und wässrigem Fluid.

Das erfindungsgemäße System bzw. die erfindungsgemäße Vorrichtung ist besonders geeignet zur Bestimmung des Verockerungsrisikos von Wasser, wässrigen Fluiden und/oder wasserhaltigen Systemen. Weiterhin kann Korrosion oder Rostbildung in diesen Systemen detektiert werden.

Das erfindungsgemäße System bzw. die erfindungsgemäße Vorrichtung kann weiterhin eine Ausgabeeinheit umfassen. Diese Ausgabeeinheit ist insbesondere so ausgestaltet, dass das Verockerungsrisiko des Wassers oder des wässrigen Fluids, insbesondere des wasserhaltigen Systems numerisch und/oder farblich codiert dargestellt wird. Eine entsprechende Codierung kann anzeigen, dass das Verockerungsrisiko gering, durchschnittlich oder hoch ist. Insbesondere aufbauend auf den hier vorliegend genannten Bereichen kann die Ausgabeeinheit ausgestaltet sein, um das Verockerungsrisiko numerisch und/oder farblich codiert darzustellen.

Besonders bevorzugt handelt es sich bei der erfindungsgemäßen Vorrichtung bzw. dem erfindungsgemäßen System um eines ausgebildet als mobiles Handgerät und/oder eingerichtet zur Echtzeit und/oder kontinuierlichen Messung der Bildungsrate von $Fe^{3+}$ und/oder $Fe^{3+}$-Hydroxid und einer entsprechenden Darstellung des Verockerungsrisikos.

Die erfindungsgemäße Vorrichtung bzw. das erfindungsgemäße System ist insbesondere eines ausgebildet zur Durchführung des erfindungsgemäßen Verfahrens.

Das erfindungsgemäße Verfahren und die erfindungsgemäße Vorrichtung bzw. das erfindungsgemäße System erlauben das Verockerungsrisiko sowohl biotischer als auch abiotischer Verockerung einzuschätzen.

Das erfindungsgemäße Verfahren ist eines, bei dem die Bildungsrate von $Fe^{3+}$ und/oder $Fe^{3+}$-Hydroxid in Wasser oder wässrigen Fluid aus einer Korrektur des Redoxpotentials von $Fe^{2+}$/ $Fe^{3+}$ mit einem Faktor bestimmt aus dem Sauerstoffgehalt $O_2$ des Wassers, berechnet wird. Es zeigte sich, dass die Bildungsrate von $Fe^{3+}$ und/oder $Fe^{3+}$-Hydroxid in Wasser bzw. wässrigem Fluid proportional zum Sauerstoffgehalt $O_2$ des Wassers ist. Weiterhin ist der Einfluss des Sauerstoffs auf die Platinelektrodenoberfläche logarithmisch. Entsprechend kann das Redoxpotential mit dem $O_2$-Gehalt korrigiert werden. Dieses wird beispielhaft in den sich anschließenden Beispielen dargestellt.

Durch das erfindungsgemäße Verfahren bzw. unter Einsatz der erfindungsgemäßen Vorrichtung bzw. des erfindungsgemäßen Systems ist eine Früherkennung von wasserchemischen Veränderungen mit Verockerungsrisiko möglich und erlaubt somit eine frühzeitige Prävention. Eine solche Vorrichtung, die als Sensor ausgebildet sein kann, benötigt einen geringen Wartungsaufwand. Im Wesentlichen genügt es, den redoxsensitiven Elektrodenteil eines Handgeräts nach der Messung zu reinigen sowie eine einfache Kalibrierung der pHsensitiven Elektrode sowie der sauerstoffsensitiven Elektrode vorzunehmen. Entsprechend können einfache Feldmessgeräte hergestellt werden.

Erfindungsgemäß sind aber auch fest installierte Vorrichtungen und Systeme, wie Sensoren möglich. Solche fest installierten Sensoren können insbesondere auf dem Gebiet der wasserhaltigen Systeme insbesondere der offenen und geschlossenen wasserhaltigen Systems eingesetzt werden.

Mit Hilfe des erfindungsgemäßen Verfahrens und der erfindungsgemäßen Vorrichtungen bzw. Systeme ist es unter Nutzung der Redoxelektrode im bestimmten Redoxpotential $Fe^{2+}$/ $Fe^{3+}$ in Kombination mit den gemessenen Aktivitäten des gelösten Sauerstoffs und in Verbindung mit dem gemessenen pH-Wert sowie der Temperatur möglich, die Bildungsrate von $Fe^{3+}$ bzw. $Fe^{3+}$-Hydroxid zu bestimmen, um so eine kostengünstige und schnelle Beurteilung von Wasser bzw. wässrigen Fluiden zu erlauben.

Insbesondere durch eine Onlineüberwachung, d.h. eine Echtzeitüberwachung ist es möglich Anlagenstörungen in entsprechenden hydraulischen Systemen zu verhindern. Brunnenregenerationen lassen sich besser planen und die Regenerationsintervalle optimieren. In Anlagen, in denen Korrosionsinhibitoren eingesetzt werden, lassen sich die Funktionen ebenfalls überwachen und eine entsprechende Korrosion frühzeitig erkennen. Einsparungen in der Menge der eingesetzten Korrosionsschutzmittel lassen sich so realisieren, ebenso die Reduzierung bzw. einen effektiveren Einsatz von chemischen Mitteln zur Regenerierung der Brunnen. Dieses hat sowohl ökologische als auch ökonomische Vorteile. Insbesondere ist ein Einsatz in geothermischen Systemen und Kühlsystemen, die auf der Nutzung natürlicher Grundwasser beruhen, ist möglich. Bei Niedertemperaturheizanlagen, wie Betonkernaktivierung kann die Funktionsfähigkeit verbessert und der Energieverbrauch der Heizungsanlagen verringert werden.

Insgesamt ergeben sich ein geringerer Wartungsaufwand, eine höhere Betriebssicherheit und damit reduzierte ökonomische und ökologische Kosten und Aufwendungen zur Sicherstellung und Bewahrung der Funktionsfähigkeit entsprechender hydraulischer Anlagen. Im Folgenden wird der herausgefundene Zusammenhang zur Berechnung der Eisengeschwindigkeit auf Basis des bestimmten Redoxpotentials, pH-Werts, Sauerstoffgehalts und Temperatur dargestellt:

Grundsätzlich errechnet sich die Eisen (II)-Ionenaktivität in M (Mol*Liter$^{-1}$) aus dem gemessenen und auf die Standardwasserstoffelektrode normierten sowie elektrodenspezifisch bezüglich des Sauerstoffeinflusses auf die Messung gemäß Opel, O., et al., 2008, Vom Wasser 106(4), 14-21 und eines ebenfalls elektrodenspezifischen Offset korrigierte Redoxpotential $E_h$'/mV mit pH-Wert und Temperatur wie folgt:

$$a\left(Fe^{2+}\right) = 10\wedge((E_0 - E_h{}') / E_N + \log\left(K_s^*\right) - 3 \cdot pH) \qquad / \mathrm{M} \qquad (1)$$

Darin ist

$$E_N = \ln 10 \cdot R \cdot \frac{T}{F} \quad / \text{V} \qquad (2)$$

mit

R= allg. Gaskonstante
F = Faraday Konstante
T = absolute Temperatur in K
und $E_0$ =0,771 V

sowie:

$$\log K_s^*(Fe(OH)_{3(s)}) = \log K_s(Fe(OH)_{3(s)}) + n \cdot pK_w \qquad (3)$$

mit n: stöchiometrischer Faktor bzgl. der Bildung von $Fe(OH)_n^{(3-n)}$, hier n=3, und:

$$\begin{aligned} \ln K_w &= 148{,}9802 - 13847{,}26/T - 23{,}6521 \cdot \ln T \\ &+ 2 \cdot \left[ I^{0,5} / \left(1 + 1{,}2 \cdot I^{0,5}\right) + 1{,}667 \cdot \ln\left(1 + 1{,}2 \cdot I^{0,5}\right) \right] \\ &\cdot \left(-5{,}8901 + 228{,}2338/T + 0{,}968144 \cdot \ln T\right) \\ &- I \cdot \left(20{,}6365 - 945{,}556/T - 3{,}00298 \cdot \ln T\right) \\ &- 0{,}18062 \cdot \left[1 - \left(1 + 2 \cdot I^{0,5} - 2 \cdot I\right) \cdot \exp\left(-2 \cdot I^{0,5}\right)\right] \\ &- I^2 \cdot \left(-0{,}05346 + 17{,}6216/T\right) \end{aligned} \qquad (4)$$

Sowie:

$$\log K_s(Fe(OH)_{3(s)}) = -115{,}14 + 23{,}75/T + 13{,}76 \cdot \ln(T) \qquad (5)$$

Die Berechnung der Geschwindigkeit der Verockerung durch die Bildung von $Fe(OH)_3$ (Bildungsrate von $Fe^{3+}$ bzw.Eisen(III)hydroxid) geschieht aus der Eisen(II)-Ionenaktivität sowie aus pH-Wert, Sauerstoffgehalt und der Temperatur

Die Geschwindigkeit der $Fe(OH)_3$-Bildung $v = \frac{d(Fe(OH)_3)}{dt}$ in $M{*}min^{-1}$ errechnet sich mit

$$\frac{d(Fe(OH)_3)}{dt} = k \cdot a\left(Fe^{2+}\right) \cdot p\left(O_2\right) \cdot a\left(OH^-\right)^2 \quad / \text{M*min}^{-1} \qquad (6)$$

und

$$k \sim 10^{14}\ \text{M}^{-2}\text{atm}^{-1}\text{min}^{-1} \qquad (7)$$

Die $OH^-$ - Aktivität errechnet sich nach

$$pOH = pK_w - pH \qquad (8)$$

Dabei ist bei der Berechnung des $pK_w$ entsprechend Gleichung (4) zu beachten.
Der Einfluss der Temperatur auf die Geschwindigkeitskonstante k der Gesamtreaktion

$$4\ Fe^{2+}_{(aq)} + O_2 + 10\ H_2O \rightleftharpoons 4\ Fe(OH)_{3(s)} + 8\ H^+ \qquad (9)$$

ergibt sich, wenn gleichzeitig die Effekte auf den $pK_w$ beachtet werden, mit

$$\frac{\delta \ln k}{\delta T} = \frac{\Delta H_1^{\ddagger}}{RT^2} \qquad (10)$$

mit $\Delta H_1^{\ddagger}$ = 29 ± 2kJ*mol$^{-1}$.

Im Folgenden wird das erfindungsgemäße Verfahren am Beispiel des Aquifer-Energiespeichers des Energiesystems der Parlamentbauten am Berliner Spreebogen dargestellt.

Dabei wurden in den Jahren 2000 bis 2009 vor Ort routinemäßig die Parameter Redoxpotential, Sauerstoffpartialdruck, pH-Wert, Leitfähigkeit und Temperatur bestimmt. Unter Anwendung des erfindungsgemäßen Verfahrens konnte dabei die Bildungsrate von $Fe^{3+}$ bzw. $Fe^{3+}$-Hydroxid im Wasser der Aquiferen bestimmt werden. Entsprechend der oben beschriebenen Methode und Berechnung konnten die in der Abbildung 1 dargestellten Ergebnisse erhalten werden. Figur 1 zeigt die Bildungsgeschwindigkeit von $Fe^{3+}$ in den Aquiferspeicherwässern. KS PLH bedeutet der Kältespeicher, WS PLH der Wärmespeicher des Berliner Reichstags.PLH bezeichnet dabei die Probennahmestelle der Filtertöpfe im Paul-Löbe-Haus. Deutlich sind die unterschiedlichen Bildungsgeschwindigkeiten zu erkennen. Mit Hilfe der Pfeile sind notwendige Filterwechsel z. B. aufgrund stark belegter Filter (Verockerung) dargestellt.

Gleiches wird deutlich aus der Figur 2. Dort sind die Eisen- und Schwefelgehalte vor und hinter den Filtersäcken im Vergleich dargestellt. Bei oxidierenden Verhältnissen wird aus in Wasser gelöstem $Fe^{2+}$ unlösliches $Fe(OH)_3$ gebildet, das ausgefällt wird. Entsprechend ergeben sich geringere Eisengehalte nach Filtration als vor Filtration (negative Werte), wenn das Verockerungsrisiko hoch ist. Nach Desinfektion des Systems fand zunächst kein Ausfall von Eisen (III)-Hydroxid statt. Entsprechend sind die Werte positiv, d.h. im Filter vorhandenes Eisen wird durch Reduktion wieder in Lösung überführt.

Gegen Ende 2008 zeigten sich ca. 0,2 g/m$^3$ * min (entsprechend ca. 10%) geringere Eisengehalte im Wasser hinter den Filtern als vor den Filtern, was bei Zugrundelegung der Oxidationsraten aus Abbildung 1 (ca. 0,02 g/m$^3$ * min) während dieser Zeit stark auf eine Verockerung hinweist Bei einem Anlagenvolumen von ca. 15 m$^3$ und einer Durchflussrate von ca. 100 m$^3$/h entsprechend 1,66 m$^3$/min. ergibt sich bei einer sich daraus ergebenden Aufenthaltszeit des Wassers in der Anlage vor der Filtereinrichtung von ca. 9 min. eine Bildung von ca. 0,18 g/m$^3$, was die gemessenen Werte von ausgefälltem, im Filter zurückgehaltenen Eisen somit gut quantitativ erklären kann.

Weiterhin konnten gemessene Filterbelegungen mit Eisen und Schwefel durch vorhergegangene starke, erfindungsgemäß berechnete Eisenhydroxid-Bildungsraten erklärt werden (eine Anlagerung von Schwefel in den Filtern geschieht als Folge von verstärkter Eisenoxidation, die die Milieubedingungen für eine verstärkte Schwefelreduktion unter Bildung unlöslicher und daher in den Filtern abgeschiedener Sulfidverbindungen schafft). Figur 3 zeigt mit den geleisteten Betriebsstunden der Filter korrigierte Anreicherungsfaktoren von Eisen und Schwefel. Die beobachteten Phänomene können durch vorhergehend erfindungsgemäß bestimmte $Fe^{3+}$- bzw. Eisenhydroxid-Bildungsraten erklärt werden.

Erfindungsgemäß kann also unter Anwendung des vorliegenden Verfahrens bzw. unter Verwendung eines entsprechenden Systems bzw. einer erfindungsgemäßen Vorrichtung die Bildungsrate von $Fe^{3+}$ und/oder $Fe^{3+}$-Hydroxid bestimmt werden, um so direkt eine Aussage über das Verockerungsrisiko des Wassers bzw. des wässrigen Fluids im betrachteten hydraulischen System zu erlauben.

## Patentansprüche

1. Verfahren zur Bestimmung der Bildungsrate von $Fe^{3+}$ und/oder $Fe^{3+}$-Hydroxid im Wasser oder wässrigen Fluid, umfassend die Schritte:

   a) Bestimmen des Redoxpotentials $Fe^{2+}$/ $Fe^{3+}$ mit Hilfe einer Redoxelektrode,
   b) Bestimmen des pH-Wertes,
   c) Bestimmen der Temperatur, und
   d) Bestimmen des Sauerstoffgehalts

des Wassers oder des wässrigen Fluids und Berechnung der Bildungsrate gemäß der Gleichung (6)

$$\frac{d(Fe(OH)_3)}{dt} = k \cdot a(Fe^{2+}) \cdot p(O_2) \cdot a(OH^-)^2 \qquad / \ M^*min^{-1}$$

in Verbindung mit der Gleichung (1)

$$a(Fe^{2+}) = 10\wedge((E_0 - E_h')/E_N + \log(K_s^*) - 3 \cdot pH) \qquad / \ M$$

und wobei k~$10^{14}$ $M^{-2}$ $atm^{-1}$ $min^{-1}$ ist, mit $\log K_s^*(Fe(OH)_{3(s)}) = \log K_s(Fe(OH)_{3(s)}) + n \cdot pKw$ mit n = 3, wobei das Bestimmen der Bildungsrate von $Fe^{3+}$ und/oder $Fe^{3+}$-Hydroxid im Wasser oder wässrigen Fluid die Korrektur des Redoxpotentials von $Fe^{2+}$/ $Fe^{3+}$ mit einem Faktor, bestimmt aus dem Sauerstoffgehalt $O_2$ des Wassers, beinhaltet

**2.** Verfahren nach Anspruch 1 weiterhin umfassend mindestens einen der Schritte:

- Bestimmen der $Fe^{2+}$ Konzentration,
- Bestimmen der $Fe^{3+}$ Konzentration,
- Bestimmen der $Fe^{2+}$ Aktivität,
- Bestimmen des $CO_2$-Gehalts, und/oder
- Bestimmen der Leitfähigkeit

des Wassers oder wässrigen Fluids.

**3.** Verfahren nach einem der Ansprüche 1 oder 2 weiterhin umfassend mindestens einen der Schritte:

- Bestimmen des Gehalts an Nitrat, Phosphat, organischem Kohlenstoff und/oder Carbonat
- Bestimmen der Turbidität
- Bestimmen der Färbung.

**4.** Verfahren nach einem der vorherigen Ansprüche zur, gegebenenfalls kontinuierlichen und/oder Echtzeit-, Bestimmung des Verockerungsrisikos des Wassers oder wässrigen Fluids, insbesondere in einem wasserhaltigen System.

**5.** Verfahren nach einem der vorherigen Ansprüche zur Bestimmung des Verockerungsrisikos in geschlossenen, wasserhaltigen Systemen.

**6.** Verfahren nach einem der vorherigen Ansprüche, wobei dieses erlaubt, dass Verockerungsrisiko einer biotischen oder abiotischen Verockung einzuschätzen.

**7.** Verfahren nach einem der vorherigen Ansprüche, wobei das Verockerungsrisiko sehr groß ist, wenn die Bildungsrate von $Fe^{3+}$ und/oder $Fe^{3+}$-Hydroxid $\geq$ 0,1 $g/m^{3*}$min ist, das Verockerungsrisiko groß ist, wenn die Bildungsrate von $Fe^{3+}$ und/oder $Fe^{3+}$-Hydroxid < 0,1 $g/m^{3*}$min und $\geq$ 0,01 $g/m^{3*}$min ist, es durchschnittlich ist, wenn die Bildungsrate von $Fe^{3+}$ und/oder $Fe^{3+}$-Hydroxid < 0,01 $g/m^{3*}$min und $\geq$ 0,001 $g/m^{3*}$min ist und das Verockerungsrisiko gering ist, wenn die Bildungsrate von $Fe^{3+}$ und/oder $Fe^{3+}$-Hydroxid < 0,001 $g/m^{3*}$min ist.

**8.** Vorrichtung oder System zur Bestimmung der Bildungsgeschwindigkeit von $Fe^{3+}$ und/oder $Fe^{3+}$-Hydroxid in Wasser oder wässrigen Fluids umfassend eine Einrichtung zur Bestimmung des Redoxpotenzials von $Fe^{2+}$/ $Fe^{3+}$, die eine Redoxelektrode umfasst; eine Einrichtung zur Bestimmung des Sauerstoffgehalts im Wasser oder wässrigen Fluid; eine Einrichtung zur Bestimmung der Temperatur des Wassers oder des wässrigen Fluids; und eine Einrichtung zur Bestimmung des pH-Werts des Wassers oder wässrigen Fluids, wobei diese Vorrichtung oder dieses System ausgebildet ist zur Durchführung eines Verfahrens nach einem der Ansprüche 1 bis 7.

**9.** Vorrichtung oder System nach Anspruch 8, weiterhin umfassend mindestens eine Einrichtung zur Bestimmung mindestens eines der folgenden Parameter: $Fe^{2+}$-Konzentration, $Fe^{2+}$-Aktivität, $CO_2$-Konzentration, Leitfähigkeit, und/oder $Fe^{3+}$-Konzentration des Wassers oder wässrigen Fluids.

**10.** Vorrichtung oder System nach Anspruch 8 oder 9, weiterhin umfassend eine Auswerteeinheit zum Bestimmen der Bildungsgeschwindigkeit von $Fe^{3+}$ und/oder $Fe^{3+}$-Hydroxid im Wasser oder wässrigem Fluid.

**11.** Vorrichtung oder System nach einem der Ansprüche 8 bis 10 zur Bestimmung des Verockerungsrisikos von Wasser, wässrigen Fluiden und/oder wasserhaltigen Systemen.

**12.** Vorrichtung oder System nach einem der Ansprüche 8 bis 11, weiterhin umfassend eine Ausgabeeinheit, insbesondere eine Ausgabeeinheit, die das Verockerungsrisiko des Wassers oder der wässrigen Fluids, insbesondere des wasserhaltigen Systems numerisch und/oder farblich codiert darstellt.

**13.** Vorrichtung oder System nach einem der Ansprüche 8 bis 12, ausgebildet als mobiles Handgerät.

**14.** Vorrichtung oder System nach einem der Ansprüche 8 bis 12, eingerichtet zur Echtzeit- und/oder kontinuierlichen Messung.

**Claims**

**1.** Method for determining the rate of formation of $Fe^{3+}$ and/or $Fe^{3+}$ hydroxide in water or an aqueous fluid, comprising the following steps:

a) determination of the redox potential $Fe^{2+}/Fe^{3+}$ by means of a redox electrode,
b) determination of the pH,
c) determination of the temperature and
d) determination of the oxygen content of the water or of the aqueous fluid and calculation of the rate of formation according to the equation (6)

$$\frac{d(Fe(OH)_3)}{dt} = k \cdot a(Fe^{2+}) \cdot p(O_2) \cdot a(OH^-)^2 \qquad / \mathrm{M^*min^{-1}}$$

in conjunction with the equation (1)

$$a(Fe^{2+}) = 10\text{^}((E_0 - E_h')/E_N + \log(K_s^*) - 3 \cdot pH) \qquad / \mathrm{M}$$

and where $k \sim 10^{14}\ M^{-2}\ atm^{-1}\ min^{-1}$
and $\log K_s^*(Fe(OH)_{3(s)}) = \log K_s(Fe(OH)_{3(s)}) + n.pKw$ with $n = 3$, where the determination of the rate of formation of $Fe^{3+}$ and/or $Fe^{3+}$ hydroxide in water or an aqueous fluid comprises correction of the redox potential of $Fe^{2+}/Fe^{3+}$ by a factor determined from the oxygen content $O_2$ of the water.

**2.** Method according to Claim 1, further comprising at least one of the following steps:

- determination of the $Fe^{2+}$ concentration,
- determination of the $Fe^{3+}$ concentration,
- determination of the $Fe^{2+}$ activity,
- determination of the $CO_2$ content and/or
- determination of the conductivity

of the water or the aqueous fluid.

**3.** Method according to either of Claims 1 and 2, further comprising at least one of the following steps:

- determination of the content of nitrate, phosphate, organic carbon and/or carbonate,
- determination of the turbidity,
- determination of the colour.

**4.** Method according to any of the preceding claims for determining, optionally continuously and/or in real time, the risk of formation of iron-containing precipitates in the water or aqueous fluid, in particular in a water-containing system.

**5.** Method according to any of the preceding claims for determining the risk of formation of iron-containing precipitates in closed, water-containing systems.

**6.** Method according to any of the preceding claims, wherein the method allows the risk of biotic or abiotic formation of iron-containing precipitates to be estimated.

**7.** Method according to any of the preceding claims, wherein the risk of formation of iron-containing precipitates is very high when the rate of formation of $Fe^{3+}$ and/or $Fe^{3+}$ hydroxide is ≥ 0.1 g/m$^3$*min, the risk of formation of iron-containing precipitates is high when the rate of formation of $Fe^{3+}$ and/or $Fe^{3+}$ hydroxide is < 0.1 g/m$^{3*}$min and ≥ 0.01 g/m$^3$*min, is average when the rate of formation of $Fe^{3+}$ and/or $Fe^{3+}$ hydroxide is < 0.01 g/m$^{3*}$min and ≥ 0.001 g/m$^{3*}$min and the risk of formation of iron-containing precipitates is low when the rate of formation of $Fe^{3+}$ and/or $Fe^{3+}$ hydroxide is < 0.001 g/m$^3$*min.

**8.** Apparatus or system for determining the speed of formation of $Fe^{3+}$ and/or $Fe^{3+}$ hydroxide in water or an aqueous fluid, comprising a device for determining the redox potential of $Fe^{2+}/Fe^{3+}$ which comprises a redox electrode; a device for determining the oxygen content in the water or aqueous fluid; a device for determining the temperature of the water or of the aqueous fluid; and a device for determining the pH of the water or aqueous fluid, where this apparatus or this system is configured for carrying out a method according to any of Claims 1 to 7.

**9.** Apparatus or system according to Claim 8, which further comprises at least one device for determining at least one of the following parameters: $Fe^{2+}$ concentration, $Fe^{2+}$ activity, $CO_2$ concentration, conductivity and/or $Fe^{2+}$ concentration of the water or aqueous fluid.

**10.** Apparatus or system according to Claim 8 or 9, which further comprises an evaluation unit for determining the speed of formation of $Fe^{3+}$ and/or $Fe^{3+}$ hydroxide in the water or the aqueous fluid.

**11.** Apparatus or system according to any of Claims 8 to 10 for determining the risk of formation of iron-containing precipitates in water, aqueous fluids and/or water-containing systems.

**12.** Apparatus or system according to any of Claims 8 to 11, which further comprises an output unit, in particular an output unit which displays, in numerical and/or colour-coded form, the risk of formation of iron-containing precipitates in the water or the aqueous fluid, in particular the water-containing system.

**13.** Apparatus or system according to any of Claims 8 to 12, configured as mobile hand-held instrument.

**14.** Apparatus or system according to any of Claims 8 to 12, equipped for real-time and/or continuous measurement.

## Revendications

**1.** Procédé de détermination du taux de formation de $Fe^{3+}$ et/ou d'hydroxyde de $Fe^{3+}$ dans de l'eau ou un fluide aqueuse, comprenant les étapes suivantes :

a) la détermination du potentiel redox de $Fe^{2+}/Fe^{3+}$ à l'aide d'une électrode redox,
b) la détermination du pH,
c) la détermination de la température, et
d) la détermination de la teneur en oxygène de l'eau ou du fluide aqueux, et le calcul du taux de formation selon l'équation (6) :

$$\frac{d(Fe(OH)_3)}{dt} = k \cdot a(Fe^{2+}) \cdot p(O_2) \cdot a(OH^-)^2 \qquad / \text{M*min}^{-1}$$

conjointement avec l'équation (1) :

$$a(Fe^{2+}) = 10\wedge((E_0 - E_h')/E_N + \log(K_s^*) - 3 \cdot pH) \qquad / M$$

et avec k ~ $10^{14}$ $M^{-2}$ $atm^{-1}$ $min^{-1}$,
avec $\log K_s^*(Fe(OH)_{3(s)}) = \log K_s(Fe(OH)_{3(s)}) + n \cdot pKw$ avec n = 3, la détermination du taux de formation de $Fe^{3+}$ et/ou d'hydroxyde de $Fe^{3+}$ dans l'eau ou le fluide aqueux comprenant la correction du potentiel redox de $Fe^{2+}/Fe^{3+}$ avec un facteur déterminé à partir de la teneur en oxygène $O_2$ de l'eau.

**2.** Procédé selon la revendication 1, comprenant en outre au moins une des étapes suivantes :

- la détermination de la concentration en $Fe^{2+}$,
- la détermination de la concentration en $Fe^{3+}$,
- la détermination de l'activité de $Fe^{2+}$,
- la détermination de la teneur en $CO_2$ et/ou
- la détermination de la conductivité

de l'eau ou du fluide aqueux.

**3.** Procédé selon l'une quelconque des revendications 1 ou 2, comprenant en outre au moins une des étapes suivantes :

- la détermination de la teneur en nitrate, phosphate, carbone organique et/ou carbonate,
- la détermination de la turbidité,
- la détermination de la coloration.

**4.** Procédé selon l'une quelconque des revendications précédentes pour la détermination, éventuellement continue et/ou en temps réel, du risque de formation d'ocre ferreux de l'eau ou du fluide aqueux, notamment dans un système contenant de l'eau.

**5.** Procédé selon l'une quelconque des revendications précédentes pour la détermination du risque de formation d'ocre ferreux dans des systèmes fermés contenant de l'eau.

**6.** Procédé selon l'une quelconque des revendications précédentes, dans lequel celui-ci permet d'évaluer le risque de formation d'ocre ferreux biotique ou abiotique.

**7.** Procédé selon l'une quelconque des revendications précédentes, dans lequel le risque de formation d'ocre ferreux est très élevé lorsque le taux de formation de $Fe^{3+}$ et/ou d'hydroxyde de $Fe^{3+}$ est ≥ 0,1 $g/m^3$*min, le risque de formation d'ocre ferreux est élevé lorsque le taux de formation de $Fe^{3+}$ et/ou d'hydroxyde de $Fe^{3+}$ est < 0,1 $g/m^3$*min et ≥ 0,01 $g/m^3$*min, il est moyen lorsque le taux de formation de $Fe^{3+}$ et/ou d'hydroxyde de $Fe^{3+}$ est < 0,01 $g/m^3$*min et ≥ 0,001 $g/m^3$*min, et le risque de formation d'ocre ferreux est faible lorsque le taux de formation de $Fe^{3+}$ et/ou d'hydroxyde de $Fe^{3+}$ est < 0,001 $g/m^3$*min.

**8.** Dispositif ou système pour la détermination de la vitesse de formation de $Fe^{3+}$ et/ou d'hydroxyde de $Fe^{3+}$ dans de l'eau ou un fluide aqueux, comprenant un appareil pour la détermination du potentiel redox de $Fe^{2+}/Fe^{3+}$, qui comprend une électrode redox ; un appareil pour la détermination de la teneur en oxygène dans l'eau ou le fluide aqueux ; un appareil pour la détermination de la température de l'eau ou du fluide aqueux ; et un appareil pour la détermination du pH de l'eau ou du fluide aqueux, ce dispositif ou ce système étant configuré pour la réalisation d'un procédé selon l'une quelconque des revendications 1 à 7.

**9.** Dispositif ou système selon la revendication 8, comprenant en outre au moins un appareil pour la détermination d'au moins un des paramètres suivants : la concentration en $Fe^{2+}$, l'activité de $Fe^{2+}$, la concentration en $CO_2$, la conductivité et/ou la concentration en $Fe^{3+}$ de l'eau ou du fluide aqueux.

**10.** Dispositif ou système selon la revendication 8 ou 9, comprenant en outre une unité d'exploitation pour la détermination de la vitesse de formation de $Fe^{3+}$ et/ou d'hydroxyde de $Fe^{3+}$ dans l'eau ou le fluide aqueux.

**11.** Dispositif ou système selon l'une quelconque des revendications 8 à 10 pour la détermination du risque de formation d'ocre ferreux d'eau, de fluides aqueux et/ou de systèmes contenant de l'eau.

**12.** Dispositif ou système selon l'une quelconque des revendications 8 à 11, comprenant en outre une unité de sortie, notamment une unité de sortie qui représente de manière codée numériquement et/ou par couleur le risque de formation d'ocre ferreux de l'eau ou du fluide aqueux, notamment du système contenant de l'eau.

**13.** Dispositif ou système selon l'une quelconque des revendications 8 à 12, configuré sous la forme d'un appareil portatif.

**14.** Dispositif ou système selon l'une quelconque des revendications 8 à 12, conçu pour la mesure en temps réel et/ou continue.

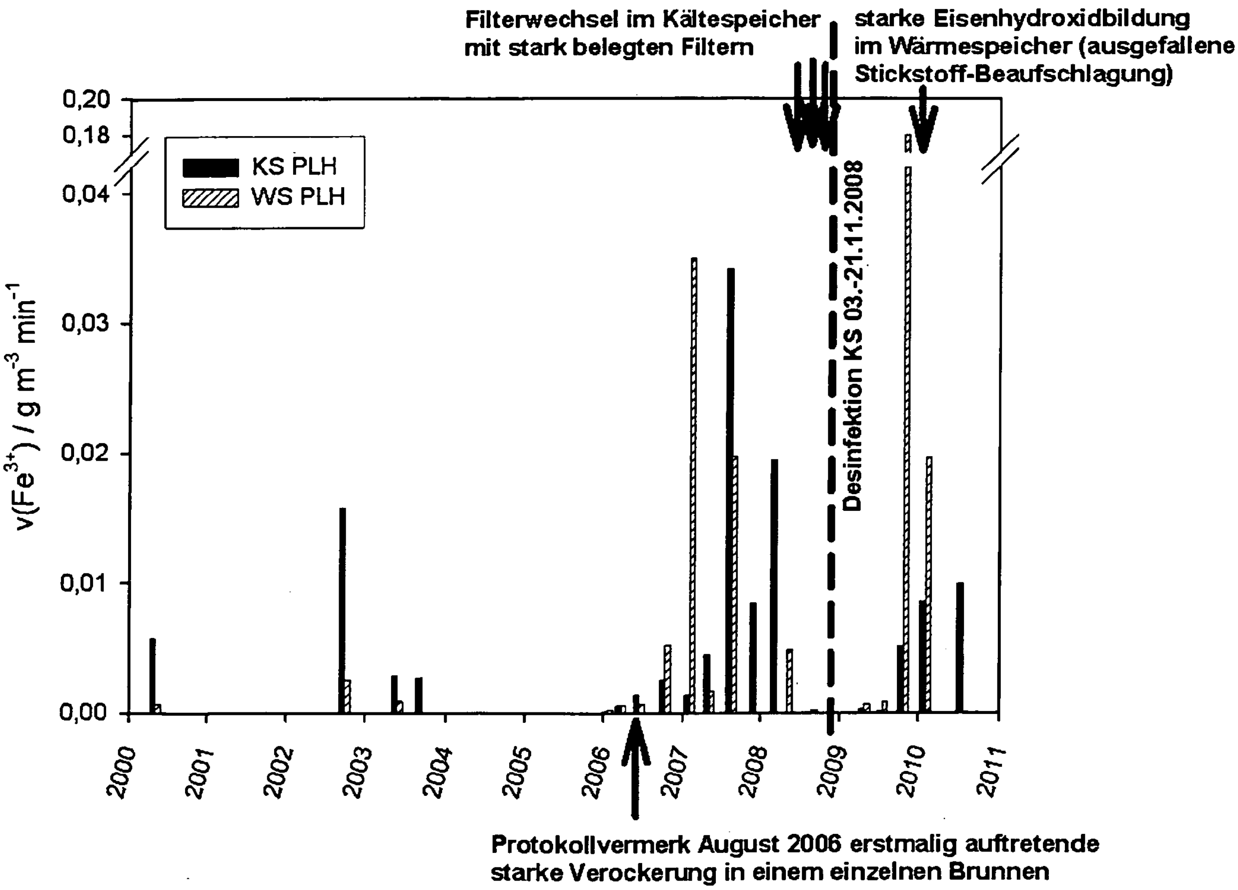
Filterwechsel im Kältespeicher mit stark belegten Filtern
starke Eisenhydroxidbildung im Wärmespeicher (ausgefallene Stickstoff-Beaufschlagung)
KS PLH
WS PLH
v(Fe3+) / g m-3 min-1
0,20
0,18
0,04
0,03
0,02
0,01
0,00
Desinfektion KS 03.-21.11.2008
2000
2001
2002
2003
2004
2005
2006
2007
2008
2009
2010
2011
Protokollvermerk August 2006 erstmalig auftretende starke Verockerung in einem einzelnen Brunnen

Figur 1

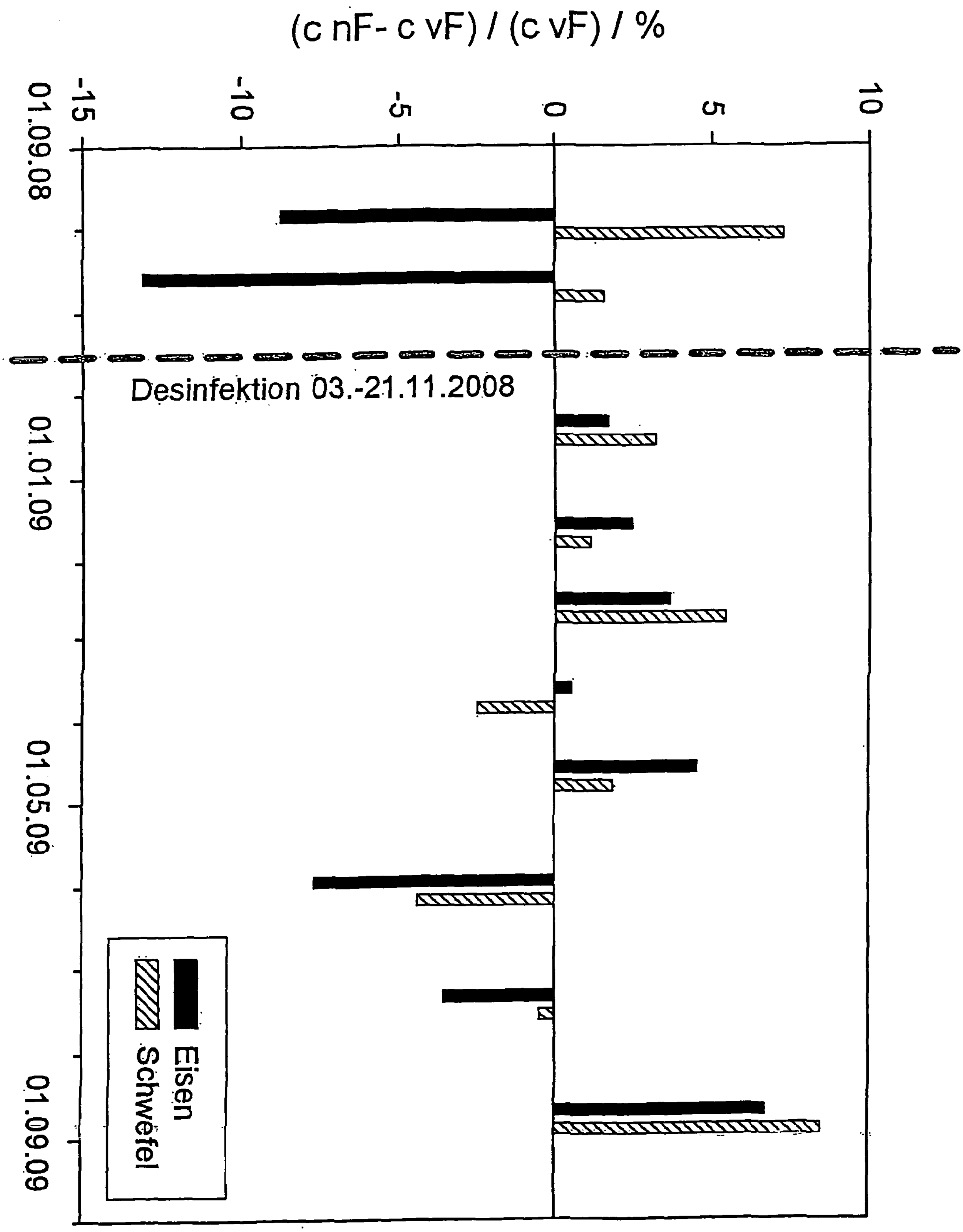
(c nF- c vF) / (c vF) / %
-15
-10
-5
0
5
10
01.09.08
01.01.09
01.05.09
01.09.09
Desinfektion 03.-21.11.2008
Eisen
Schwefel

Figur 2

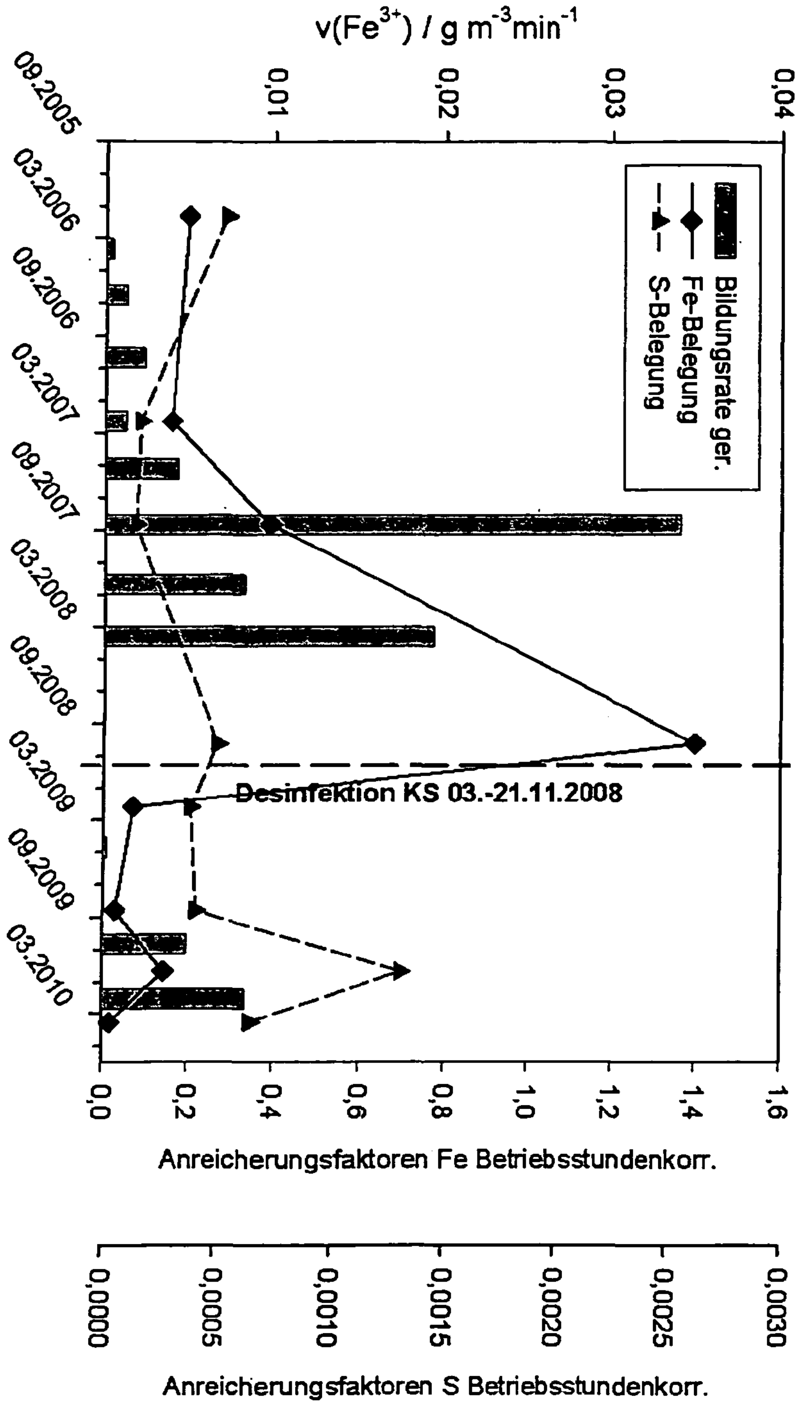
v(Fe3+) / g m-3min-1
0,01
0,02
0,03
0,04
Bildungsrate ger.
Fe-Belegung
S-Belegung
09.2005
03.2006
09.2006
03.2007
09.2007
03.2008
09.2008
03.2009
09.2009
03.2010
Desinfektion KS 03.-21.11.2008
0,0
0,2
0,4
0,6
0,8
1,0
1,2
1,4
1,6
Anreicherungsfaktoren Fe Betriebsstundenkorr.
0,0000
0,0005
0,0010
0,0015
0,0020
0,0025
0,0030
Anreicherungsfaktoren S Betriebsstundenkorr.

Figur 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **OPEL, O. et al.** *Vom Wasser,* 2008, vol. 106 (4), 14-21
- **CRAVOTTA.** *Mine Water Environ,* 2007, vol. 26, 128-149